# EUROPEAN PATENT APPLICATION

(11) **EP 3 821 922 A1**
(43) Date of publication of application: **19.05.2021**
(21) Application number: 19208922.5
(22) Date of filing: 13.11.2019
(51) Int. Cl.: A61M 1/06

(54) **BREAST PUMP SYSTEM**

(71) Applicant: Koninklijke Philips N.V., 5656 AG Eindhoven (NL)
(72) Inventor: KOSTER, Aafje Gijsbertha, 5656 AE Eindhoven (NL); VAN DE WASSENBERG, Wilhelmina Johanna Gerarda, 5656 AE Eindhoven (NL); SLOOTS, Ingrid, 5656 AE Eindhoven (NL); SANDBERG, Emma, 5656 AE Eindhoven (NL)
(74) Representative: Philips Intellectual Property & Standards

(57) **Abstract**

The invention provides a breast pump system having a belt for wearing by the user of the system. The belt has a pocket for receiving a pump of the system. The pocket has a lateral pocket opening, with a conduit extending from the pump laterally out of the pocket opening. This makes a more comfortable fitting of the pump and easier connection to the expression kit.

## Description

### FIELD OF THE INVENTION

This invention relates to breast pump systems.

### BACKGROUND OF THE INVENTION

There is a desire for mothers to be able to operate breast pumps in a hands free manner, so that they may perform other activities at the same time as performing breast pumping.

A breast pump system typically comprises an expression body comprising a shield for application to the breast and a pump for applying a pressure cycle to the expression body. A milk receptacle, such as a milk feeding bottle, is connectable to the expression body to collect the expressed milk.

Normally one or both shields are connected to the pump using air tubes, and have to be held by hand to the respective breast. The pump is for example either placed on a table or put into a suitably large pocket of a garment.

It is known to provide a pumping/feeding bra for wearing by the user of the system to avoid the need for the user to hold the interface cups by hand. Also, integrated solutions have been proposed that provide wearable storage both for the expression kit as well as for the pump body, and in some cases the milk collection receptacle.

It is also known to provide a belt for wearing by the user of the system, wherein the belt comprises one or more pockets to hold the various parts of the system. US 2016/0067393 is one example.

The pump is for example mounted vertically in a pocket in the belt. This may not be comfortable when the user is sitting and may not provide a free path for the air tubes.

There is therefore a need for a system which is more comfortable for the user and enables hands free operation, both while sitting and standing.

### SUMMARY OF THE INVENTION

The invention is defined by the claims.

According to examples in accordance with an aspect of the invention, there is provided a breast pump system, comprising:
an expression body comprising a shield for application to the breast;
a pump for applying a pressure cycle to the expression body, wherein the pump comprises a housing having an end face with a conduit interface;
a conduit between the conduit interface of the pump housing and the expression body;
a milk receptacle connectable to the expression body; and
a belt for wearing by the user of the system, wherein the belt comprises a pocket adapted to receive the pump and having a lateral pocket opening, with the conduit extending from the conduit interface of the pump housing laterally out of the pocket opening.

The use of a lateral pocket means the conduit extends laterally out of the pump (i.e. in a direction parallel to the elongate axis of the belt) rather than vertically upwards. This means it is easier to sit down and for the conduit to find a natural path between the pump and the expression kit.

The belt can be worn either way up, so that the lateral pocket opening may face forwards when the pocket is at the left side of a user or at the right side of the user. In this way, the pump can be positioned for most comfortable use for a left-handed user, or for a right-handed user.

The belt is preferably continuously adjustable so that it may be fitted to any user. It may be elastic, and may have a buckle, or other type of connector such as a hook and loop type fastener such as Velcro (Trade Mark). The belt may be made of a textile material or a neoprene type of material.

The pocket is for example rectangular, and the longest dimension of the rectangle is parallel to an elongate axis of the belt. The pocket thus extends around the belt, with a smaller height than the width around the belt. This provides a comfortable positioning of the pump, which allows the user to sit and stand.

The pump for example comprises a user interface, and the pocket comprises a window for providing access to the user interface. Thus, the user may operate the pump while it is in the pocket. The window may be an opening in the pocket or a transparent or semitransparent portion of the pocket.

The pocket may comprise an upper web and a lower web which can be coupled together around the pump. Thus, instead of sliding the pump in the pocket, the pocket may be formed around the pump. This may be an easier way to position and fix the pump, in that the desired position is selected (to make the pump comfortable and the conduit follow a clear path), and the pocket is then formed around the pump. The pump may thus be positioned from the top or from the side.

The upper web and lower web may be connectable by Velcro, or other clip or fastening.

In one set of examples, the milk receptacle may be directly attachable to the expression body. Thus, the positioning of the expression body also secures the milk receptacle in place. A pumping/feeding bra may for example be provided for holding the combination of the expression body and milk receptacle in place.

In another set of examples, the milk receptacle is attachable to the expression body through a second conduit and the belt comprises a second pocket adapted to receive the milk receptacle. This reduces the weight supported at the breast. An extra pocket may also be provided to hold a power pack to supply the breast pump with energy, or a mobile device that may be connected to the breast pump for controlling and/or monitoring purposes.

In all cases, the milk receptacle is "fluidly connectable" to the expression body and not necessarily directly mechanically connectable. The term "connectable" should be understood accordingly.

The system may then further comprise a pumping/feeding bra for holding (only) the expression body in place.

The invention also provides a belt adapted for use as the belt of the breast pump system as defined above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a first example of a breast pump system;
Figure 2 shows an example of the belt design;
Figure 3 shows a second example of a breast pump system; and
Figure 4 shows how the belt may be used either way up.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a breast pump system having a belt for wearing by the user of the system. The belt has a pocket for receiving a pump of the system. The pocket has a lateral pocket opening, with a conduit extending from the pump laterally out of the pocket opening. This makes a more comfortable fitting of the pump and easier connection to the expression kit.

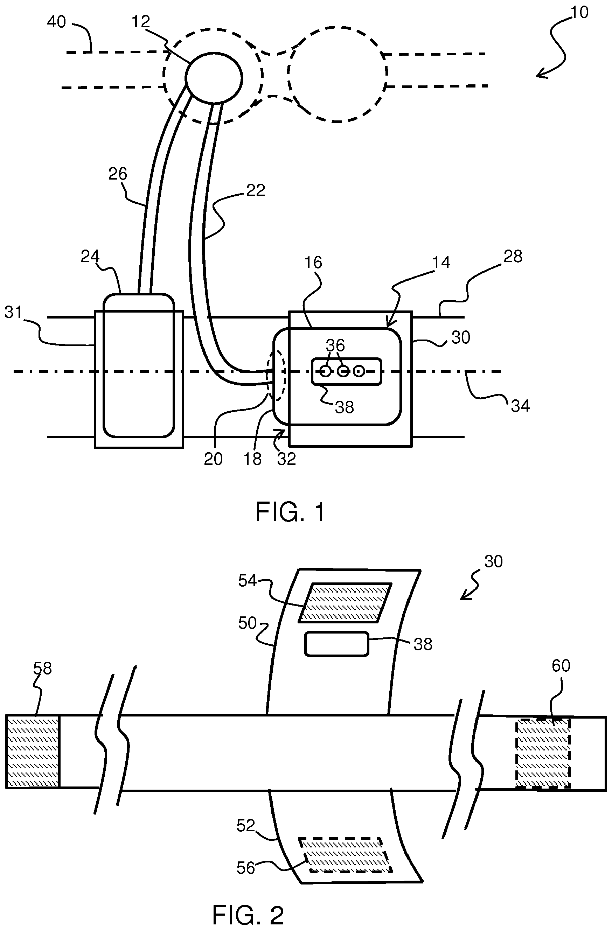

Figure 1 shows a first example of a breast pump system 10.

The system has an expression body 12 comprising a shield for application to the breast. A pump 14 is for applying a pressure cycle to the expression body 12. The pump comprises a housing 16 having an end face 18 with a conduit interface 20.

The pump is for example designed to provide a balanced push-pull vacuum that applies a pumping force to the expression body 12. The expression body in one example comprises a bellows structure which defines a vacuum chamber with a breast interface cup, during inward and outward pump strokes.

A conduit 22 extends between the conduit interface 20 of the pump housing 16 and the expression body 12.

A milk receptacle 24 is connectable to the expression body 12. In this example, the milk receptacle is connected by a second conduit 26 to the expression body 12 to collect expressed milk under gravity.

A belt 28 is provided for wearing by the user of the system.

The belt comprises a pocket 30 for receiving the pump 14. It has a lateral pocket opening 32. The (first) conduit 22 extends from the conduit interface 20 of the pump housing laterally out of the pocket opening.

In this example, the belt has a second pocket 31 for receiving the milk vessel, in a vertical orientation.

The use of a lateral pocket for the pump means the conduit extends laterally out of the pump (i.e. in a direction parallel to an elongate axis 34 of the belt 28), rather than vertically upwards. In this way, it is easier to sit down and for the conduit to find a natural path between the pump and the expression kit.

In particular, a single bend may be formed in the conduit to reach from the conduit interface to the expression body 12 (as shown), The conduit for example connects substantially vertically (e.g. with 25 degrees each side of vertical) to the expression body so that a single bend of near 90 degrees may be formed in the conduit 22.

The belt is preferably continuously adjustable so that it may be fitted to any user. It may be elastic, and may have a buckle, or other type of connector such as a Velcro connection. The belt may be made of a textile material.

The pocket 30 is for example rectangular, and the longest dimension of the rectangle is preferably parallel to an elongate axis 34 of the belt 28. The pocket thus extends around the belt, with a smaller height than the width around the belt. This provides a comfortable positioning of the pump, which allows the user to sit and stand.

The pump 14 has a corresponding rectangular shape and thus extends with its long axis around the belt direction rather than vertically up or down. The position of the conduit interface 20 on the pump housing may determine the best direction of the pocket. The pocket may be angled with respect to the horizontal rather than perfectly aligned vertically. For example, a detachable pocket may be fastened in different positions. Thus, the user could determine the best position even more easily.

Even if freely positionable, the pocket is positionable with a lateral pocket opening. By lateral is meant offset to the vertical, for example by more than 45 degrees, for example by more than 60 degrees, for example by more than 80 degrees.

The pocket may be formed of elastic material, but non-elastic straps may be used. The pocket may instead be formed as a more rigid casing.

The pump and belt pocket for example each have an aspect ratio of more than 1.5, for example more than 2.

In this example, the pump has user interface 36 for operating the pump, and the pocket has a window 38 for providing access to the user interface. Thus, the user may operate the pump while it is in the pocket. The window may be an opening in the pocket or a transparent portion of the pocket.

The pocket may be fixed, and the user slides the pump laterally into and out of the pocket. It may be open at both ends or it may be closed at one end so the pump can be inserted until it reaches the end of the pocket cavity.

The expression body 12 may be held in place against the user's breast by a pumping/feeding bra 40.

Figure 2 show a design of the belt in which the pocket 30 can be opened and closed. The pocket has an upper web 50 and a lower web 52 which can be coupled together around the pump. Thus, instead of sliding the pump in the pocket, the pocket may be formed around the pump. This may be an easier way to position and fix the pump, in that the desired position is selected (to make the pump comfortable and the conduit follow a clear path), and the pocket is then formed around the pump. The pump may thus be positioned from the top or from the side.

The upper web and lower web may be connectable by Velcro, or other clip or fastening. Figure 2 shows Velcro pads 54, 56 (one on the inside surface and one on the outside surface) which overlap when the webs are overlapped.

The ends of the belt also comprise Velcro pads 58, 60 in this example, but any belt connection may be used. The belt may be elastic or non-elastic.

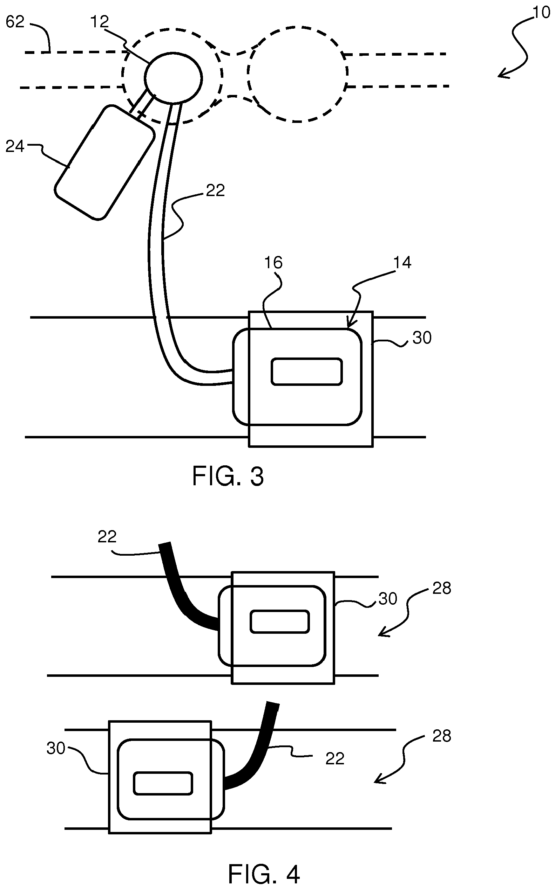

Figure 3 shows a second example of a breast pump system. In this example, the milk receptacle 24 is directly attached to the expression body and located adjacent the breast. The direct connection between the expression body 12 and the receptacle 24 may still include a conduit, but of minimal length and for example forming an integrated part of the expression body. The conduit would then be less than 10cm in length, whereas the conduit 22 is more than 10 cm long, for example in the range 30cm to 60cm in length.

Thus, the positioning of the expression body for the example of Figure 3 also secures the milk receptacle in place. A pumping/feeding bra 62 may for example be provided for holding the combination of the expression body and milk receptacle in place.

Figure 4 shows how the belt 28 may be used either way up so that the lateral opening of the pocket can be made to face in either direction around the belt, i.e. clockwise around the waist of the user or counterclockwise around the waist of the user. The lateral pocket opening may then be oriented to face forwards when the pocket is at the left side of a user or at the right side of the user. In this way, the pump can be positioned for most comfortable use for a left-handed user, or for a right-handed user.

The pump may create a pressure cycle by using overpressure, or else by mechanically creating the pressure cycle.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A breast pump system (10), comprising:
an expression body (12) comprising a shield for application to the breast;
a pump (14) for applying a pressure cycle to the expression body, wherein the pump comprises a housing (16) having an end face (18) with a conduit interface (20);
a conduit (22) between the conduit interface of the pump housing (16) and the expression body (12);
a milk receptacle (24) connectable to the expression body (12); and
a belt (28) for wearing by the user of the system, wherein the belt comprises a pocket (30) adapted to receive the pump and having a lateral pocket opening (32), with the conduit (22) extending from the conduit interface (20) of the pump housing laterally out of the pocket opening.

2. A system as claimed in claim 1, wherein the pocket (30) is rectangular, and the longest dimension of the rectangle is parallel to an elongate axis (34) of the belt.

3. A system as claimed in claim 1 or 2, wherein the pump (14) comprises a user interface (36) and the pocket comprises a window (38) for providing access to the user interface.

4. A system as claimed in claim 3, wherein the window (38) is an opening in the pocket or a transparent portion of the pocket.

5. A system as claimed in any one of claims 1 to 4, wherein the pocket (30) comprises an upper web (50) and a lower web (52) which can be coupled together around the pump.

6. A system as claimed in claim 5, wherein the upper web and lower web are connectable by Velcro (54,56).

7. A system as claimed in any one of claims 1 to 6, wherein the milk receptacle (24) is directly attachable to the expression body (12).

8. A system as claimed in claim 7, further comprising a pumping/feeding bra (62) for holding the expression body (12) and milk receptacle (24) in place.

9. A system as claimed in any one of claims 1 to 6, wherein the milk receptacle (24) is attachable to the expression body (12) through a second conduit (26) and wherein the belt (28) comprises a second pocket (31) adapted to receive the milk receptacle.

10. A system as claimed in claim 9, further comprising a pumping/feeding bra (40) for holding the expression body in place.

11. A belt adapted for use as the belt of the breast pump system as claimed in any one of claims 1 to 10.
